# EUROPÄISCHE PATENTSCHRIFT

(11) **EP 0 912 612 B1**
(45) Veröffentlichungstag und Bekanntmachung des Hinweises auf die Patenterteilung: **01.10.2003**
(21) Anmeldenummer: 96927598.1
(22) Anmeldetag: 25.07.1996
(51) Int. Cl.: C07K 16/46

(54) **VEREINFACHTE HERSTELLUNG BISPEZIFISCHER ANTIKÖRPERFRAGMENTE**
SIMPLIFIED PRODUCTION OF BISPECIFIC ANTIBODY FRAGMENTS
PRODUCTION SIMPLIFIEE DE FRAGMENTS D'ANTICORPS BISPECIFIQUES

(43) Veröffentlichungstag der Anmeldung: 06.05.1999
(73) Patentinhaber: GSF - Forschungszentrum für Umwelt und Gesundheit, GmbH, D-85758 Oberschleissheim (DE)
(72) Erfinder: JUNG, Gundram, D-82110 Germering (DE)
(74) Vertreter: VOSSIUS & PARTNER
(86) Internationale Anmeldenummer: EP9603275
(87) Internationale Veröffentlichungsnummer: WO98004592

(56) Entgegenhaltungen:
- EP-A- 0 446 071
- EP-A- 0 453 082
- WO-A-93/13805
- WO-A-95/05399
- EUR.J.IMMUNOL., Bd. 21, 1991, Seiten 2431-2435, XP000647636 JUNG ET AL.: "target cell-induced T cell activation with bi- and trispecific antibody fragments" in der Anmeldung erwähnt
- CRITICAL REVIEWS IN IMMUNOLOGY, Bd. 12, Nr. 3/04, 1.Januar 1992, Seiten 101-124, XP000578127 FANGER M W ET AL: "BISPECIFIC ANTIBODIES"
- BEHRING INST. MITT., Nr. 78, 1985, Seiten 118-132, XP002028195 PAULUS: "Preparation and biomedical applications of bispecific antibodies"
- SCIENCE, Bd. 229, 5.Juli 1989, Seiten 81-83, XP002028196 BRENNAN ET AL.: "Preparation of bispecific antibodies by chemical recombination of monoclonal immunoglobulin G1 fragments" in der Anmeldung erwähnt
- Römmp Chemie Lexikon, 9. Auflage, S.4121 & 4393, (1992)

## Beschreibung

Die Erfindung betrifft die Herstellung von bispezifischen Antikörperfragmenten für die Immuntherapie von Tumoren in Menschen und Tieren.

Die Aktivierung des körpereigenen Immunsystems gegen bösartige Erkrankungen ist eine alte therapeutische Zielvorstellung, die allerdings bisher nicht realisiert werden konnte. In den letzten Jahren ist es aber gelungen, die Voraussetzungen dafür entscheidend zu verbessern. Die Schlüsselstrukturen für die Aktivierung des Immunsystems auf der Oberfläche von T-Zellen wurden identifiziert und charakterisiert. Dabei handelt es sich im wesentlichen um den antigen-erkennenden T-Zell Rezeptor/CD3 Komplex und das CD28-Molekül als wahrscheinlich wichtigsten kostimulierenden Rezeptor.
Es ist möglich, bispezifische Antikörper so zu konstruieren, daß sie mit der einen Hälfte spezifisch an Tumorzellen binden und mit der anderen diese zentralen "molekularen Schalter" des Immunsystems aktivieren. Die aktivierten Zellen sind in der Lage, die Tumorzellen effektiv abzutöten (Jung und Müller-Eberhard, Immunol. Today 9 (1988), 257).

Mittlerweile ist die Anti-Tumorwirkung bispezifischer Antikörper in zahlreichen *in vitro* Versuchsanordnungen und auch in mehreren Maus-Tumor-Modellen gezeigt worden (Übersicht in Beun et al., Immunol. Today 21 (1994), 2413).

Bei ersten *lokalen* Anwendungen am Menschen konnte das peritoneale Wachstum von Ovarialkarzinomen und Glioblastomen günstig beeinflußt werden (Nitta et al., Lancet 335 (1990), 368; Canevari et al., JNCI 87 (1995), 1463).
Es besteht damit die begründete Aussicht, daß mit bispezifischen Antikörpern eine effektive Immuntherapie menschlicher Tumore zumindest in bestimmten klinischen Situationen möglich sein wird.

Um eine unspezifische Aktivierung der Immunzellen über den Fc-Teil der verwendeten Antikörper zu verhindern, ist die Verwendung von bispezifischen Antikörperfragmenten ohne Fc-Teil notwendig. Im Stand der Technik wurde von Brennan et al. (Science 229 (1985), 81-83) ein Verfahren zur Herstellung von bispezifischen Antikörperfragmenten beschrieben, wobei intakte Antikörper durch Pepsin-Spaltung zu F(ab')₂-Fragmenten fragmentiert wurden, und die entstandenen F(ab')₂-Fragmente durch Säulenchromatographie gereinigt wurden. Anschließend wurden die Disulfidbrücken der Hinge-Region des gereinigten F(ab')₂-Moleküls in Gegenwart von Arsenit durch Reduktion gespalten und die so erhaltenen F(ab')-SH-Fragmente wiederum durch Säulenchromatographie gereinigt, um anschließend die reduzierten SH-Gruppen mit dem Ellmans Reagens (DTNB) zu F(ab')-TNB zu modifizieren. Nach erneuter Säulenchromatographie wurde eines der beiden Antikörperfragmente zu F(ab')-SH reduziert, über Säulenchromatographie gereinigt und mit dem anderen F(ab')-TNB-Fragment zu einem bispezifischen F(ab')₂-Fragment hybridisiert. Schließlich wurden die so erhaltenen bispezifischen Antikörperfragmente über Gelchromatographie gereinigt.

Im Stand der Technik wurde von Jung et al. (Eur. J. Immunol. 21 (1991), 2431-2435) eine Modifikation des oben genannten Verfahrens beschrieben, in dem die Spaltung der Disulfidbrücken in der Hinge-Region des F(ab')₂-Moleküls und die Blockierung der entstandenen SH-Gruppen mit einer Schutzgruppe gleichzeitig durchgeführt wurden, so daß ein Reinigungsschritt des in Brennan et al. beschriebenen Verfahrens entfiel. Trotzdem ist auch bei diesem Verfahren der Aufwand, der für die Herstellung bispezifischer Antikörperfragmente betrieben werden muß, so groß, daß bisher nicht die Substanzmengen produziert werden können, die für eine systemische Applikation bei Mensch und Tier notwendig wären. Dadurch wurde die klinische Erprobung dieser vielversprechenden Reagentien entscheidend behindert. Die Entwicklung eines Verfahrens, das die Herstellung bispezifischer Antikörperfragmente weiter vereinfacht, würde daher die Bereitstellung größerer Mengen solcher bispezifischer Antikörperfragmente und deren Einsatz in der Immuntherapie von Tumoren ermöglichen.

Aufgabe der vorliegenden Erfindung war daher, die vorstehend beschriebenen Nachteile aus dem Stand der Technik zu überwinden und ein vereinfachtes Verfahren zur Herstellung von bispezifischen Antikörperfragmenten bereitzustellen.

Die Lösung dieser Aufgabe wird durch die in den Ansprüchen gekennzeichneten Ausführungsformen erreicht.

Überraschenderweise wurde nämlich gefunden, daß die Reduktion und Modifikation eines nach Pepsinspaltung erhaltenen Antikörperfragments in einem Schritt ohne den im Stand der Technik bis dahin als notwendig erachteten Reinigungsschritt durchgeführt werden kann. Dieser Befund erlaubt eine geringere Zahl von Verfahrensschritten für die Herstellung von bispezifischen Antikörperfragmenten, wobei dieses Verfahren zusätzlich durch eine höhere Gesamtausbeute des gewünschten Endprodukts gekennzeichnet ist. Das erfindungsgemäße Verfahren wird hier für die Herstellung von bispezifischen F(ab')₂-Fragmenten aus intakten Antikörpern beschrieben. Grundsätzlich ist dieses Verfahren für die Kopplung verschiedener Proteine, sofern sie Disulfidbrücken enthalten, die durch TNB reduziert werden können, anwendbar.

Die Erfindung betrifft somit ein Verfahren zur Herstellung von bispezifischen Proteinen, vorzugsweise Antikörperfragmenten, das dadurch gekennzeichnet ist, daß bis dahin als notwendig erachtete Reinigungsschritte entfallen und dadurch das gesamte Verfahren wesentlich vereinfacht und kostengünstig gestaltet werden kann. In einer bevorzugten Ausführungsform des erfindungsgemäßen Verfahrens umfaßt die Herstellung biologisch aktiver, bispezifischer Antikörperfragmente dabei im wesentlichen die folgenden Schritte:
(a) Unterwerfen eines ersten und eines zweiten Antikörpers einem Fragmentierungsschritt unter Bedingungen, die zur Abspaltung des Fc-Teils der Antikörper und zur Bildung von F(ab')₂-Molekülen genügen;
(b) Unterwerfen der jeweiligen Produkte aus Schritt (a) ohne zwischengeschalteten Reinigungsschritt einem Reduktions- und Modifikationsschritt unter Bedingungen, die zur Spaltung der Disulfidbrücken in der Hinge-Region des F(ab')₂-Moleküls genügen und zur gleichzeitigen Blockierung der entstandenen SH-Gruppen mit einer Schutzgruppe führen;
(c) Unterwerfen der jeweiligen Produkte aus Schritt (b) einem Reinigungsschritt unter Bedingungen, die zur Entfernung der Reaktionsaddukte genügen;
(d) Unterwerfen des Produkts des ersten Antikörpers aus Schritt (c) einem Reduktionsschritt unter Bedingungen, die zur Abspaltung-der Schutzgruppe aus Schritt (b) genügen;
(e) Unterwerfen des Produkts aus Schritt (d) einem Reinigungsschritt entsprechend dem Schritt (c); und
(f) Unterwerfen des Produkts des zweiten Antikörpers aus Schritt (c) und des Produkts des ersten Antikörpers aus Schritt (e) einem Hybridisierungsschritt unter Bedingungen, die zur Bildung von bispezifischen F(ab')₂-Molekülen genügen.

Die in Schritt (a) eingesetzten intakten Antikörper können nach vorbekannten im Stand der Technik beschriebenen Methoden gewonnen werden (Current Protocols in Immunology, J. E. Codigan, A. M. Krvisbeck, D. H. Margulies, E. M. Shevack, W. Strober eds., John Wiley + Sons). Die Durchführung der einzelnen Reaktions- und Reinigungsschritte ist ebenfalls aus dem Stand der Technik bekannt und beispielsweise in Brennan et al. (Science 229 (1985), 81-83) und Jung et al. (Eur. J. Immunol. 21 (1991), 2431-2435) beschrieben.

In einer bevorzugten Ausführungsform des erfindungsgemäßen Verfahrens wird Schritt (c) bei gleichzeitiger Umpufferung auf pH 8.0 und/oder Schritt (e) bei gleichzeitiger Umpufferung auf pH 4.0 durchgeführt.

In einer ebenfalls bevorzugten Ausführungsform des erfindungsgemäßen Verfahrens wird das Produkt aus Schritt (f) einem Proteinabtrennschritt unter Bedingungen, die zur Entfernung nicht hybridisierter F(ab')-Moleküle führen, unterworfen.

In einer weiteren bevorzugten Ausführungsform werden die vorstehend beschriebenen Verfahren so durchgeführt, daß die erhaltenen Antikörperfragmente steril und/oder pyrogenfrei und/oder im wesentlichen frei von aktiven Viren sind. Diese Ausführungsform bietet sich an, wenn die bispezifischen Antikörperfragmente für die Anwendung an Patienten verwendet werden sollen. Diese Ausführungsform ist z. B. nicht unbedingt notwendig, wenn die bispezifischen Antikörperfragmente für diagnostische Zwecke vorgesehen sind.

In einer besonders bevorzugten Ausführungsform ist mindestens einer der Antikörper ein Antikörper, der Oberflächenantigene von T-Zellen erkennt.

In einer weiteren besonders bevorzugten Ausführungsform der vorgenannten Verfahren ist mindestens einer der Antikörper ein Antikörper, der Oberflächenantigene von Tumorzellen erkennt.

Durch das erfindungsgemäße Verfahren können nunmehr bispezifische Antikörper in genügenden Mengen und zu akzeptablen Kosten zur Verfügung gestellt werden, so daß die Therapie von Tumoren möglich wird, bei denen die konventionellen Methoden bisher unbefriedigend sind. Als ein bekanntes Beispiel sei das lymphatisch metastasierende maligne Melanom genannt, für dessen erfolgreiche Behandlung bisher weder immuntherapeutische noch konventionelle Therapiemöglichkeiten zur Verfügung stehen.

Somit ist eine weitere bevorzugte Ausführungsform des erfindungsgemäßen Verfahrens dadurch gekennzeichnet, daß der Oberflächenantigene von Tumorzellen erkennende Antikörper spezifisch für Melanome ist.

In einer besonders bevorzugten Ausführungsform der oben beschriebenen Verfahren ist der Oberflächenantigene von T-Zellen erkennende Antikörper spezifisch für CD3 oder CD28.

In einer weiteren besonders bevorzugten Ausführungsform ist das erfindungsgemäße Verfahren dadurch gekennzeichnet, daß die Fragmentierung in Schritt (a) die Inkubation einer Antikörperlösung für drei Stunden bei 37 °C mit Pepsin und das Abstoppen der Reaktion durch Erhöhen des pH auf 8.0 mit Tris-Puffer umfaßt.

In einer weiteren Ausführungsform des erfindungsgemäßen Verfahrens umfaßt die Reduktion und Modifikation in Schritt (b) die Zugabe eines gleichen Volumens eines DTNB-TNB-Gemischs für 20 Stunden bei Raumtemperatur.

In einer weiteren bevorzugten Ausführungsform des erfindungsgemäßen Verfahrens umfaßt der Reinigungsschritt in den Schritten (c) und (e) die Gelfiltration auf Superdex® 200 (c) und/oder Sephadex® G20-Säulen. Der Fachmann weiß aus dem Stand der Technik, wie er die Bedingungen für die Reinigung der Produkte (c) und (e) mittels der oben genannten Säulen auszuwählen hat. Zudem kann der Fachmann auch andere Verfahren zur Reinigung der Produkte aus dem erfindungsgemäßen Verfahren aus dem Stand der Technik entnehmen (Current Protocols in Immunology, J. E. Codigan, A. M. Krvisbeck, D. H. Margulies, E. M. Shevack, W. Strober eds., John Wiley + Sons).

In einer weiteren bevorzugten Ausführungsform des erfindungsgemäßen Verfahrens umfaßt der Reduktionsschritt in Schritt (d), die Inkubation des Produktes aus Schritt (c) in 0.1 mM DTT. Diese Ausführungsform ist besonders bevorzugt und verwendet im Gegensatz zu den Verfahren im Stand der Technik, siehe z.B. Brennan et al. (Science 229 (1985), 81-83) und Jung et al. (Eur. J. Immunol. 21 (1991), 2431-2435), eine sehr kleine, annähernd stöchiometrische DTT-Konzentration bezüglich der mit TNB blockierten Protein-SH-Gruppen, mit der die Wahrscheinlichkeit einer Reduktion der Disulfidbrücken zwischen schweren und leichten Ketten minimiert ist. Dadurch läßt sich nicht nur die Gesamtausbeute von bispezifischen Antikörperfragmenten durch das erfindungsgemäße Verfahren erhöhen, sondern auch die Qualität der Produkte verbessern.

In einer weiteren besonders bevorzugten Ausführungsform des Verfahrens erfolgt die Hybridisierung in Schritt (f) durch Reaktion gleicher Volumen von F(ab')-TNB in 0.1 M Phosphatpuffer pH 8.0 und F(ab')-SH in 0:02 M Acetatpuffer pH 4.0.

Die Figuren zeigen:

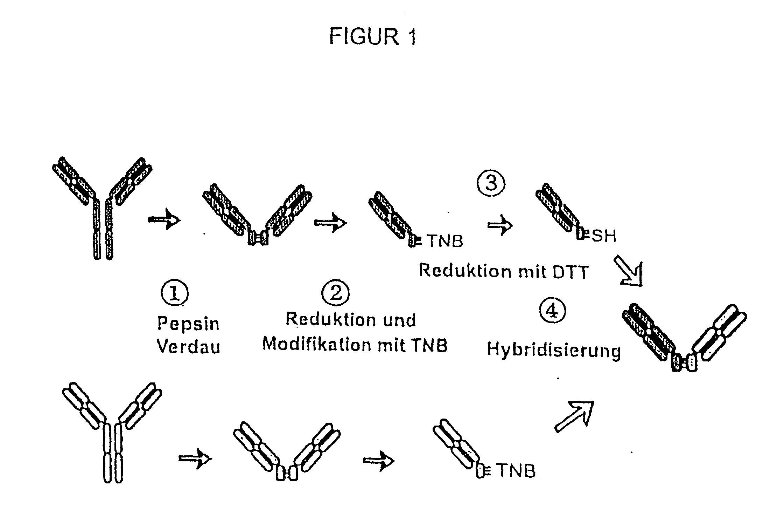

- Fig. 1: Herstellung eines bispezischen F(ab')₂-Fragments nach dem erfindungsgemäßen Verfahren.

Die Beispiele erläutern die Erfindung.

### Beispiel 1:

### Herstellung von bispezifischen F(ab')₂-Antikörperfragmenten

Ausgangspunkt für das erfindungsgemäße Verfahren sind intakte Antikörper, die, wie im Stand der Technik beschrieben (Current Protocols in Immunology, J. E. Codigan, A. M. Krvisbeck, D. H. Margulies, E. M. Shevack, W. Strober eds., John Wiley + Sons), gewonnen werden. Für dieses Beispiel wurden Antikörper benutzt, die spezifisch für ein melanomassoziiertes Proteoglycan bzw. für das humane mit dem antigenspezifischen T-Zell-Rezeptor assoziierte CD3-Molekül sind. Diese Antikörper wurden mit Pepsin (Sigma) drei Stunden bei 37°C in Acetat Puffer pH 4.0 behandelt, um den Fc-Teil des Antikörpers abzuspalten (Fig. 1(1)). Die Reaktion wurde durch Erhöhen des pH-Werts auf 8 mit Tris-Puffer abgestoppt und die resultierende Lösung mit einem gleichen Volumen eines Gemischs aus 5,5'-Dithiobis-2-Nitrobenzoesäure (DTNB; Sigma) und Thionitrobenzoat (TNB) für 20 Stunden bei Raumtemperatur inkubiert (Fig. 1(2)). Das molare Verhältnis des DTNB-TNB-Gemischs beträgt 20:30 und wird durch die Inkubation einer 40 mM DTNB-Lösung mit einer 10 mM DTT-Lösung für einige Minuten eingestellt. Nach erneuter Reduktion einer der beiden modifizierten F(ab')-Fragmente mit 0.1 mM DTT (Sigma) für eine Stunde bei 25 °C (Fig. 1(3)) werden die so erhaltenen F(ab')-TNB und F(ab')-SH-Fragmente zusammengegeben und für 1h bei 25 °C zu einem bispezifischen F(ab')₂-Fragment hybridisiert (Fig. 1(4)). Die erhaltenen bispezifischen F(ab')₂-Fragmente wurden dann durch Gelfiltration auf einer Superdex® 200-Säule gereinigt. Die Ausbeute bezüglich der Menge an eingesetzten intakten Antikörper betrug etwa 20 %. Im Vergleich zu den im Stand der Technik beschriebenen Verfahren konnte damit die Ausbeute um 50-100 % erhöht werden.

### Beispiel 2:

### Funktionalität und Stabilität der bispezifischen F(ab')₂-Fragmente

Alle verwendeten Antikörper und die damit hergestellten bispezifischen Konstrukte wurden auf ihre Funktionalität und Stabilität nach Standardverfahren (Jung et al., Eur. J. Immunol. 21 (1991), 2431-2435) getestet. Die Funktionalität und Stabilität der nach dem erfindungsgemäßen Verfahren hergestellten F(ab')₂-Fragmente war denen nach alter Methodik hergestellten vergleichbar. Dies betrifft u. a. die Bindung an menschliches Tumormaterial, die Aktivität in Lymphocytenproliferations- und Zytotoxitätstests und die Stabilität unter *in vivo* Bedingungen; inkubiert in menschlichem Serum bei 37 °C waren die verwendeten Konstrukte für mindestens 6 Tage, in Phosphatpuffer bei 5 °C für mehrere Monate funktionell stabil.

### Beispiel 3:

### Test der bispezifischen F(ab')₂-Fragmente an B16 Melanomzellen in der Maus

Die nach dem erfindungsgemäßen Verfahren hergestellten bispezifische Antikörperfragmente wurden auch auf ihre in vivo Aktivität im Tierversuch getestet. Nach diesen Untersuchungen sind die vorgenannten F(ab')₂-Fragmente in der Lage, das Wachstum von Melanomzellen in Mäusen so zu hemmen, daß mehr als die Hälfte der behandelten Tiere überleben, wohingegen 100 % aller unbehandelten Tiere sterben.

## Patentansprüche

1. Verfahren zur Herstellung biologisch aktiver, bispezifischer Antikörperfragmente, umfassend die Schritte:
(a) Unterwerfen eines ersten und eines zweiten Antikörpers einem Fragmentierungsschritt unter Bedingungen, die zur Abspaltung des Fc-Teils der Antikörper und zur Bildung von F(ab')₂-Molekülen genügen;
(b) Unterwerfen der jeweiligen Produkte aus Schritt (a) ohne zwischengeschalteten Reinigungsschritt einem Reduktions- und Modifikationsschritt unter Bedingungen, die zur Spaltung der Disulfidbrücken in der Hinge-Region des F(ab')₂-Moleküls genügen und zur gleichzeitigen Blockierung der entstandenen SH-Gruppen mit einer Schutzgruppe führen;
(c) Unterwerfen der jeweiligen Produkte aus Schritt (b) einem Reinigungsschritt unter Bedingungen, die zur Entfernung der Reaktionsaddukte genügen;
(d) Unterwerfen des Produkts des ersten Antikörpers aus Schritt (c) einem Reduktionsschritt unter Bedingungen, die zur Abspaltung der Schutzgruppe aus Schritt (b) genügen;
(e) Unterwerfen des Produkts aus Schritt (d) einem Reinigungsschritt entsprechend dem Schritt (c); und
(f) Unterwerfen des Produkts des zweiten Antikörpers aus Schritt (c) und des Produkts des ersten Antikörpers aus Schritt (e) einem Hybridisierungsschritt unter Bedingungen, die zur Bildung von bispezifischen F(ab')₂-Molekülen genügen.

2. Verfahren nach Anspruch 1, wobei in Schritt (c) gleichzeitig eine Umpufferung auf pH 8.0 und/oder in Schritt (e) gleichzeitig eine Umpufferung auf pH 4.0 erfolgt.

3. Verfahren nach Anspruch 1 oder 2, wobei folgender weiterer Schritt durchgeführt wird:
Unterwerfen des Produkts aus Schritt (f) einem Proteinabtrennschritt unter Bedingungen, die zur Entfernung nicht hybridisierter F(ab')-Moleküle führt.

4. Verfahren nach einem der Ansprüche 1 bis 3, wobei die erhaltenen Antikörperfragmente steril, pyrogenfrei und im wesentlichen frei von aktiven Viren sind.

5. Verfahren nach einem der Ansprüche 1 bis 4, wobei mindestens einer der Antikörper Oberflächenantigene von T-Zellen erkennt

6. Verfahren nach einem der Ansprüche 1 bis 5, wobei mindestens einer der Antikörper Oberflächenantigene von Tumorzellen erkennt

7. Verfahren nach Anspruch 6, wobei der Oberflächenantigene von Tumorzellen erkennende Antikörper spezifisch für Melanome ist.

8. Verfahren nach einem der Ansprüche 5 bis 7, wobei der Oberflächenantigene von T-Zellen erkennende Antikörper spezifisch für CD3 oder CD28 ist.

9. Verfahren nach einem der Ansprüche 1 bis 8, wobei die Fragmentierung in Schritt (a) die Inkubation einer Antikörperlösung für 3 Stunden bei 37°C mit Pepsin und das Abstoppen der Reaktion durch Erhöhen des pH auf 8.0 mit Tris-Puffer umfaßt.

10. Verfahren nach einem der Ansprüche 1 bis 9, wobei die Reduktion und Modifikation in Schritt (b) die Zugabe eines gleichen Volumens einer DTNB/TNB-Mischung für 20 Stunden bei Raumtemperatur umfaßt.

11. Verfahren nach einem der Ansprüche 1 bis 10, wobei der Reinigungsschritt in den Schritten (c) und (e) durch Gelfiltration auf Superdex® 200(c) bzw. Sephadex® G20 Säulen erfolgt.

12. Verfahren nach einem der Ansprüche 1 bis 11, wobei die Abspaltung in Schritt (d) die Inkubation des Produkts aus Schritt (c) in 0.1 mM DTT umfaßt.

13. Verfahren nach einem der Ansprüche 1 bis 12, worin die Hybridisierung in Schritt (f) durch Reaktion gleicher Volumina von F(ab')-TNB in 0.1 M Phosphatpuffer pH 8.0 und F(ab')-SH in 0.02 M Acetat-Puffer pH 4.0 erfolgt.

## Claims

1. A method for producing biologically active, bispecific antibody fragments, comprising the steps of:
(a) subjecting a first and a second antibody to a fragmentation step under conditions sufficient to cleave off the Fc portion of the antibodies and to form F(ab')₂ molecules;
(b) subjecting the respective products of step (a) without intermediate purification step to a reduction and modification step under conditions sufficient to enable cleavage of the disulfide bonds in the hinge region of the F(ab')₂ molecule and at the same time blocking the resulting SH groups with a protecting group;
(c) subjecting the respective products of step (b) to a purification step under conditions sufficient to remove the reaction adducts;
(d) subjecting the product of the first antibody of step (c) to a reduction step under conditions sufficient to cleave off the protecting group of step (b);
(e) subjecting the product of step (d) to a purification step according to step (c); and
(f) subjecting the product of the second antibody of step (c) and the product of the first antibody of step (e) to a hybridisation step under conditions sufficient to form bispecific F(ab')₂ molecules.

2. The method according to claim 1 **characterised in that** step (c) is carried out while simultaneously rebuffering the solution to pH 8.0, and/or step (e) while simultaneously rebuffering to pH 4.0.

3. The method according to claim 1 or 2 **characterised by** carrying out the following further step:
subjecting the product of step (f) to a protein separation step under conditions
resulting in the removal of non-hybridised F(ab') molecules.

4. The method according to any one of claims 1 to 3 **characterised in that** the antibody fragments obtained are sterile, pyrogen-free and essentially free of active viruses.

5. The method according to any one of claims 1 to 4 **characterised in that** at least one of the antibodies recognises surface antigens of T cells.

6. The method according to any one of claims 1 to 5 **characterised in that** at least one of the antibodies recognises surface antigens of tumour cells.

7. The method according to claim 6 **characterised in that** the antibody recognising surface antigens of tumour cells is specific for melanomas.

8. The method according to any one of claims 5 to 7 **characterised in that** the antibody recognising surface antigens of T cells is specific for CD3 or CD28.

9. The method according to any one of claims 1 to 8 **characterised in that** the fragmentation in step (a) comprises incubating an antibody solution for three hours at 37°C with pepsin and terminating the reaction by increasing the pH to 8.0 with Tris buffer.

10. The method according to any one of claims 1 to 9 **characterised in that** the reduction and modification in step (b) comprises the addition of an equal volume of a DTNB/TNB mixture for 20 hours at room temperature.

11. The method according to any one of claims 1 to 10 **characterised in that** the purification step in steps (c) and (e) is carried out by gel filtration on Superdex® 200(c) and/or Sephadex® G20 columns.

12. The method according to any one of claims 1 to 11 **characterised in that** the reduction step in step (d) comprises the incubation of the product of step (c) in 0.1 mM DTT.

13. The method according to any one of claims 1 to 12 **characterised in that** the hybridisation in step (f) is brought about by reaction of equal volumina of F(ab')-TNB in 0.1 M phosphate buffer, pH 8.0, and F(ab')-SH in 0.02 M acetate buffer, pH 4.0.

## Revendications

1. Procédé de fabrication de fragments d'anticorps bispécifiques biologiquement actifs comprenant les étapes :
(a) Soumission d'un premier et d'un deuxième anticorps à une étape de fragmentation dans des conditions suffisantes pour la séparation de la partie Fc de l'anticorps et à la formation de molécules F(ab')₂ ;
(b) Soumission des produits respectifs issus de l'étape (a), sans étape de purification intermédiaire, à une étape de réduction et de modification dans des conditions suffisantes pour la rupture des ponts disulfure dans la région charnière de la molécule F(ab')₂ et qui entraînent le blocage simultané des groupes SH obtenus avec un groupe de protection ;
(c) Soumission des produits respectifs issus de l'étape (b) à une étape de purification dans des conditions suffisantes pour l'élimination des produits réactionnels ;
(d) Soumission du produit du premier anticorps issu de l'étape (c) à une étape de réduction dans des conditions suffisantes pour la séparation du groupe de protection de l'étape (b);
(e) Soumission du produit issu de l'étape (d) à une étape de purification correspondant à l'étape (c) ; et
(f) Soumission du produit du deuxième anticorps issu de l'étape (c) et du produit du premier anticorps issu de l'étape (e) à une étape d'hybridation dans des conditions suffisantes pour la formation de molécules F(ab')₂ bispécifiques.

2. Procédé selon la revendication 1, dans lequel un changement de tampon à pH 8,0 est simultanément effectué à l'étape (c) et/ou un changement de tampon à pH 4,0 est simultanément effectué à l'étape (e).

3. Procédé selon la revendication 1 ou 2, dans lequel l'étape supplémentaire suivante est réalisée :
Soumission du produit issu de l'étape (f) à une étape de séparation de protéine dans des conditions qui entraînent l'élimination des molécules F(ab') non hybridées.

4. Procédé selon l'une des revendications 1 à 3, dans lequel les fragments d'anticorps obtenus sont stériles et/ou exempts de pyrogènes et/ou pour l'essentiel exempts de virus actifs.

5. Procédé selon l'une des revendications 1 à 4, dans lequel au moins l'un des anticorps est un anticorps qui reconnaît les antigènes de surface des cellules T.

6. Procédé selon l'une des revendications 1 à 5, dans lequel au moins l'un des anticorps est un anticorps qui reconnaît les antigènes de surface des cellules tumorales.

7. Procédé selon la revendication 6, dans lequel l'anticorps qui reconnaît les antigènes de surface des cellules tumorales est spécifique des mélanomes.

8. Procédé selon l'une des revendications 5 à 7, dans lequel l'anticorps qui reconnaît les antigènes de surface des cellules T est spécifique des CD3 ou CD28.

9. Procédé selon l'une des revendications 1 à 8, dans lequel la fragmentation dans l'étape (a) comprend l'incubation d'une solution d'anticorps pendant 3 heures à 37 °C avec de la pepsine et l'arrêt de la réaction en augmentant le pH à 8,0 avec un tampon Tris.

10. Procédé selon l'une des revendications 1 à 9, dans lequel la réduction et la modification dans l'étape (b) comprennent l'ajout d'un volume égal d'un mélange DTNB-TNB pendant 20 heures à température ambiante.

11. Procédé selon l'une des revendications 1 à 10, dans lequel l'étape de purification des étapes (c) et (e) comprend la filtration sur gel sur des colonnes Superdex® 200 (c) et/ou Sephadex® G20.

12. Procédé selon l'une des revendications 1 à 11, dans lequel la réduction dans l'étape (d) comprend l'incubation du produit issu de l'étape (c) dans 0,1 mM de DTT.

13. Procédé selon l'une des revendications 1 à 12, dans lequel l'hybridation dans l'étape (f) s'effectue par une réaction de volumes égaux de F(ab')-TNB dans 0,1 M d'un tampon phosphate à pH 8,0 et de F(ab')-SH dans 0,02 M d'un tampon acétate à pH 4,0.
